# EUROPEAN PATENT APPLICATION

(11) **EP 2 246 065 A1**
(43) Date of publication of application: **03.11.2010**
(21) Application number: 09005948.6
(22) Date of filing: 29.04.2009
(51) Int. Cl.: A61K 38/48, A61P 25/14, A61P 25/16

(54) **Intrastriatal botulinum toxin therapy**

(71) Applicant: Merz Pharma GmbH & Co. KGaA, 60318 Frankfurt am Main (DE)
(72) Inventor: Benecke, Reiner, Prof. Dr., 18147 Rosctock (DE); Wree, Andreas Prof. Dr., 18107 Elmenhorst (DE); Mix, Eilhard Dr., 18057 Rostock (DE)
(74) Representative: Ricker, Mathias

(57) **Abstract**

*Clostridium botulinum* toxin for the treatment of a movement disorder caused by or associated with hyperactive inhibitory cholinergic neurons of the striatum of a mammal, comprising administering an effective amount to said mammal, wherein the toxin is administered into striatal tissue of the mammal's brain.

## Description

### FIELD OF THE INVENTION

The present invention relates to methods for treating movement disorders, especially Parkinson's disease, by administering a *Clostridium botulinum* toxin, wherein the composition is administered into the striatum of a mammal's brain.

More particular, the present invention relates *Clostridium botulinum* toxin for the treatment of a movement disorder caused by or associated with hyperactive inhibitory cholinergic neurons of the striatum of a mammal, comprising administering an effective amount to said mammal, wherein the toxin is administered into striatal tissue of the mammal's brain.

### BACKGROUND OF THE INVENTION

### Clostridial toxins

Botulinum toxin is produced by the bacterium *Clostridium.* There are seven antigenically distinct serotypes of botulinum toxin, namely botulinum toxin A, B, C, D, E, F and G. Botulinum toxins, when released from lysed Clostridium cultures are generally associated with other bacterial proteins, which together form a toxin complex. The neurotoxic subunit of this complex is referred herein as the "neurotoxic component" of the botulinum toxin complex. This complex usually contains additional, so-called "non-toxic" proteins, which we will refer to as "complexing proteins" or "bacterial proteins". The complex of neurotoxic component and bacterial proteins is referred to as "*Clostridium botulinum* toxin complex" or "*botulinum* toxin complex". The molecular weight of this complex may vary from about 300,000 to about 900,000 Da. The complexing proteins are, for example, various hemagglutinins. The proteins of this toxin complex are not toxic themselves but provide stability to the neurotoxic component and are responsible for oral toxicity in botulinum intoxications. Unlike the toxin complex, the neurotoxic component in its isolated and pure form, i.e. devoid of any complexing Clostridium proteins, is acid labile and does not resist the aggressive environment in the gastrointestinal tract.

The neurotoxic component of the botulinum toxin complex is initially formed as a single polypeptide chain, having in the case of serotype A a molecular weight of approximately 150 kDa. In other serotypes the neurotoxic component has been observed to vary between about 145 and about 170 kDa, depending on the bacterial source. Proteolytic processing of the single chain polypeptide results in an activated polypeptide in the form of a dichain polypeptide, consisting of a heavy chain and a light chain, which are linked by a disulfide bond. In humans, the heavy chain mediates binding to pre-synaptic cholinergic nerve terminals and internalization of the toxin into the cell. The light chain is believed to be responsible for the toxic effects, acting as zink-endopeptidase and cleaving specific proteins responsible for membrane fusion (SNARE complex) (see e.g. Montecucco C., Shiavo G., Rosetto O: The mechanism of action of tetanus and botulinum neurotoxins. Arch Toxicol. 1996; 18 (Suppl.): 342-354)). By disrupting the process of membrane fusion within the cells, Botulinum toxins prevent the release of acetylcholine into the synaptic cleft. The overall effect of Botulinum toxin at the neuro-muscular junction is to interrupt neuro-muscular transmission, and, in effect, denervate muscles. Botulinum toxin also has activity at other peripheral cholinergic synapses, causing a reduction of salivation or sweating.

Each serotype of Botulinum toxin binds to the serotype specific receptor sites on the pre-synaptic nerve terminal. The specificity of Botulinum toxin for cholinergic neurons is based on the high affinity of the heavy chain for the receptor sites on these nerve terminals (Ref.: Katsekas S., Gremminloh G., Pich E.M.: Nerve terminal proteins; to fuse to learn. Transneuro Science1994; 17: 368-379).

Despite its toxic effects, botulinum toxin complex has been used as a therapeutic agent in a large number of diseases. Botulinum toxin serotype A was approved for human use in the United States in 1989 for the treatment of strabism, blepharospasm, and other disorders. It is commercially available as Botulinum toxin A protein complex, for example, under the tradename BOTOX (Allergan Inc) or under the tradename DYSPORT (Ipsen Ltd). The pure neurotoxic component of serotype A in a composition that is devoid of any other proteins of the *Clostridium botulinum* toxin complex is commercially available under the trade name XEOMIN from Merz Pharmaceuticals.

For therapeutic application the Botulinum toxin is injected directly into the muscle to be treated. The effect of Botulinum toxin is only temporary, which is the reason why repeated administration of Botulinum toxin may be required to maintain a therapeutic effect.

Tetanospasmin (TeNT) is the neurotoxin produced by the vegetative spore of *Clostridium tetani* in anaerobic conditions, causing tetanus. In one embodiment, the Botulinum toxin used herein is exchanged with tetanospasmin.

### Parkinson's disease

Parkinson's disease is a disorder of the brain characterized by shaking and difficulty with walking, movement and coordination. Parkinson's disease is associated with damage to a part of the brain that controls muscle movement. It may affect one or both sides of the body with varying degrees of loss of function. Parkinson's disease may also impair speech. It was first described in England in 1817 by James Parkinson.

Parkinson's disease is progressively degenerative until the patient has difficulty in dressing, shaving, bathing, writing, getting in and out of the car, and even eating. When Parkinson's disease affects the throat and neck muscles, the patient is in danger of malnutrition. Parkinson's disease is also called *paralysis agitans* and shaking palsy.

There are many symptoms of Parkinson's disease and every individual will not experience all of them. The most common symptoms of Parkinson's disease include: muscle rigidity, stiffness, difficulty bending arms or legs, unstable, stooped, or slumped-over posture, loss of balance, changes in walking pattern, shuffling walk, slow movements, difficulty beginning to walk, difficulty initiating any voluntary movement, small steps followed by the need to run to maintain balance, freezing of movement when the movement is stopped, inability to resume, movement muscle aches and pains (myalgia), shaking, tremors, changes in facial expression reduced ability to show facial expressions, unability to close mouth, reduced rate of blinking, voice/speech changes, slow speech, difficulty of speaking, loss of fine motor skills, difficulty of writing(small and illegible), difficulty of eating.

Parkinson's disease is caused by the progressive deterioration of the nerve cells of the part of the brain that controls muscle movement. The impaired parts of the brain are the basal ganglia and the extra-pyramidal area. In this condition, deterioration of this area of the brain reduces the amount of dopamine available to the body. When there is insufficient dopamine, the balance between dopamine and other neurotransmitters, such as acetylcholine becomes disturbed. Without dopamine, the nerve cells cannot properly transmit messages and a loss of muscle function occurs.

The exact reason that the cells of the brain deteriorate is unknown. The disorder may affect one or both sides of the body, with varying degrees of loss of function.

Furthermore, some patients with Parkinson's disease become severely depressed. A person with severe Parkinson's may have overall mental deterioration also. Mental deterioration includes dementia and hallucinations. Dementia may also be a side effect of some of the medications used to treat the disorder.

Parkinson's disease usually occurs in adults. It is rare in children. When Parkinson's occurs in children, the cause is usually decreased sensitivity of the nerves (post-synaptic) to dopamine and not because of deterioration of the area of the brain that produces dopamine. However, such forms of Parkinson's are also encompassed by the treatment of the present invention.

Parkinson's affects approximately 2 out of 1,000 people. Parkinson's often develops after age 50 and affects both men and women. It is one of the most common neurological disorders of the elderly.

In the past therapeutic approaches focussed on systemic anti-acetylcholinergic as well as pro-dopaminergic pharmacotherapy, which, however, is accompanied by quite severe side-effects e.g. neuropsychiatric disorders. The poor tolerance of the known medicaments makes the development of new approaches like e.g. neurosurgical deep-brain stimulation necessary.

Some years ago the inventors of WO 01/95924 already contemplated about the intracranial use of botulinum toxin for the treatment of movement disorders, *inter alia* Parkinson's disease. They envisaged, however, a treatment by administration of a botulinum toxin formulation to the *globus pallidus* or *thalamus* of the patient's brain, respectively, and the use of biodegradable polymers for the gradual release of the toxin (cf. examples 1 to 3).

It has been surprisingly found, however, that the intrastriatal (i.e. "into the striatum") injection of botulinum toxin formulations leads to an significant alleviation of the symptoms of movement disorders such as Parkinson's disease.

### OBJECTS OF THE INVENTION

The present invention relates to a new method of treating a disease or condition caused by or associated with hyperactive inhibitory cholinergic neurons in the striatum of a patient.

It is noteworthy that the concept of the present invention, which involves the administration of the botulinum toxin, generally allows the treatment of any condition which is associated with hyperactive cholinergic neurons in the striatum of a patient. Treatments offered by the present invention, however, may be directed primarily at idiopathic Parkinson's disease as well as to drug-induced Parkinson's disease and Parkinson-plus syndromes.

### SUMMARY OF THE INVENTION

The invention relates to a *Clostridium botulinum* toxin for the treatment of a movement disorder caused by or associated with hyperactive inhibitory cholinergic neurons of the striatum of a mammal, comprising administering an effective amount to said mammal, wherein the toxin is administered into striatal tissue of the mammal's brain.

In one embodiment said mammal is a human.

In one embodiment the invention relates to *Clostridium botulinum* toxin, wherein the movement disorder comprises at least one of the symptoms selected from muscle rigidity, tremor, a slowing of physical movement (bradykinesia) and a loss of physical movement (akinesia).

In another embodiment said movement disorder is Parkinson's disease.

In yet another embodiment the before mentioned striatal tissue is selected from putamen, nucleus caudatus, sensorimotor striatum and associative striatum or any combination thereof.

In yet another embodiment the before mentioned administration is split into several local and temporal or local or temporal subdivisions.

In yet another embodiment the before mentioned temporal subdivision is an administration at an interval of less than three months, the interval comprising a first injection and at least a second injection, wherein the amount administered in the second injection can be lower, higher or identical to the amount administered in the first injection.

In yet another embodiment the before mentioned *Clostridium botulinum* toxin is selected from the group consisting of type A, B, C, D, E, F, G or a mixture thereof.

In yet another embodiment the before mentioned *Clostridium botulinum* toxin is the neurotoxic component devoid of any other protein component of the *Clostridium botulinum* toxin complex.

In yet another embodiment the before mentioned neurotoxic component is serotype A.

In yet another embodiment the before mentioned administration is selected from the group of injection, perfusion and infusion including application by infusion pumps.

### DETAILED DESCRIPTION OF THE INVENTION

The present invention relates to a *Clostridium botulinum* toxin for the treatment of a movement disorder caused by or associated with hyperactive inhibitory cholinergic neurons of the striatum of a mammal, such as Parkinson's disease, comprising administering an effective amount of said toxin to said mammal, wherein the toxin is administered into striatal tissue of the mammal's brain.

The terms "*Clostridium botulinum* toxin" or "botulinum toxin" or "botulinum toxins", refer to the neurotoxic component (i.e. the protein responsible for the toxic effect in humans and animals) devoid of any other clostridial proteins, but also to the "botulinum toxin complex". It is used herein in cases when no discrimination between the two states of the neurotoxic component is necessary or desired.

Clostridium botulinum toxin is obtainable, for example, by cultivation of *Clostridium* bacteria. The *Clostridium* species of the present invention is *Clostridium botulinum.* However, it is important to note that botulinum toxin may be derived from any other bacterial species, provided it is a functional homolog of the botulinum toxin derived from *Clostridium botulinum.*

As used herein, the terms "toxin complex" or "botulinum toxin complex" or "botulinum neurotoxin complex" are interchangeable and refer to a high molecular weight complex comprising the neurotoxic component of approximately 150 kDa and, in addition, non-toxic proteins of *Clostridium* botulinum, including hemagglutinin and non-hemagglutinin proteins (Sakaguchi 1983; Sugiyama 1980).

The terms "neurotoxic component" or "neurotoxin component" as used throughout the specification, relates to the subunit of the botulinum toxin complex which has a neurotoxic activity and which has a molecular weight of approximately 150kDa in serotype A. The term "neurotoxic component" also includes the functional homologs found in the other serotypes of *Clostridium botulinum.* As mentioned above, in one embodiment of the present invention, the neurotoxic component is devoid of any other *C*. *botulinum* protein, especially also devoid of RNA potentially associated with the neurotoxic component. The neurotoxic component may be the single chain precursor protein of approximately 150kDa or the proteolytically processed neurotoxic component, comprising the light chain (L_{c}) of approximately 50kDa and the heavy chain (H_{c}) of approximately 100kDa, which may be linked by one or more disulfide bonds (for a review see e.g. Simpson LL, Ann Rev Pharmacol Toxicol. 2004; 44:167-93). Those of skill in the art will appreciate that full biological activity is attained only after proteolytic activation, even though it is conceivable that the unprocessed precursor can exert some biological functions or be partially active. "Biological activity" refers to (a) receptor binding, (b) internalization, (c) translocation across the endosomal membrane into the cytosol, and/or (d) endoproteolytic cleavage of proteins involved in synaptic vesicle membrane fusion. In vitro assays for assessing biological activity include the mouse LD₅₀ assay and the mouse hemidiaphragm assay as described by Pearce LB, Borodic GE, First ER, MacCallum RD (1994) (Measurement of botulinum toxin activity: evaluation of the lethality assay. Toxicol Appl Pharmacol 128: 69-77) and Dressler D, Lange M, Bigalke H (2005) (The mouse diaphragm assay for detection of antibodies against botulinum toxin type B. Mov Disord 20:1617-1619).

When producing the neurotoxic component, the toxin may be isolated from the bacteria as a complex containing the neurotoxic component. Subsequently, the neurotoxic component may be purified from the toxin complex.

Botulinum toxins, in particular the toxin complex described above, have previously been classified into seven serologically distinct types A, B, C, D, E, F and G. In recent years, distinct populations of the A- (A1 and A2) and C-serotypes (C1 and C2) have been identified. Herein, these populations are designated as "subtypes".

The neurotoxic component of serotype A is commercially available under the trade name XEOMIN from Merz Pharmaceuticals in a composition that is devoid of any other proteins of the *Clostridium botulinum* toxin complex.

Alternatively, the botulinum toxin used in some of the methods of the present invention may be generated by recombinant gene expression. To this end, an open reading frame encoding the neurotoxic component or a mutant thereof may be cloned into a vector adapted for gene expression in a host cell of interest. Methods for recombinant gene expression and protein purification are known to the person skilled in the art.

The recombinant nucleic acid molecule encoding the neurotoxic component may be derived from a known nucleic acid sequence or may be recombined from two or more known sequences by recombinant techniques or chemical synthesis. An example of a chimeric neurotoxic component is a molecule generated by fusing e.g. the light chain of a first serotype to the heavy chain of a second serotype of the neurotoxic component. An example of chemical synthesis is the chemical synthesis of the entire neurotoxic component.

Also included are genetically modified neurotoxic components containing 1, 2, 3, 4, 5, 6, 7, 8, 9, 10 or up to 20 amino acid mutations. A mutation may be a substitution, an insertion, an addition or a deletion. The mutation does not compromise any of the biological activities indicated above. However, it is also envisaged to use mutations to modulate the biological activity of the neurotoxic component.

In addition thereto, modified as well as recombinant produced neurotoxic components of botulinum toxins including the respective mutations, deletions, etc. are also within the scope of the present invention. With respect to suitable mutants, reference is made to WO 2006/027207 A1, WO 2006/114308 A1 and WO 2009/015840 which are fully incorporated by reference herein. Furthermore, within the present invention, mixtures of various serotypes (in the form of the botulinum toxin, the neurotoxic component or recombinant form or e.g. mixtures of botulinum toxins of types A and B) may be used. The present invention, however, also refers to botulinum toxins which are chemically modified, e.g. by pegylation, glycosylation, sulfatation, phosphorylation or any other modification, in particular of one or more surface or solvent exposed amino acid(s).

In another embodiment the botulinum toxin is a polypeptide comprising: a heavy chain-domain or fragment thereof of the neurotoxic component of a clostridial toxin; and a first light chain domain or fragment thereof, and at least one further light chain domain or fragment thereof, wherein the first and second light chain domain may be the same or different from each other. Such a polypeptide is disclosed for example in EP08015287.9 which is fully incorporated by reference herein.

Also included are botulinum toxins containing chemically modified amino acids, for example one or more amino acids which are glycosylated, acetylated or otherwise modified, which may be beneficial to the uptake or stability of the toxin. Particularly the lipidation of the neurotoxic component is envisaged. Modifying residues may be added to the botulinum toxin e.g. by means of an enzymatic *in vitro* reaction or by using appropriate chemical reaction conditions. Alternatively, modifying enzymatic functions may be provided *in trans* by expressing the enzyme within the host cell. In another embodiment the neurotoxic component is modified to reduce its immunogenicity. Such a polypeptide is disclosed for example in EP08015288.7 which is fully incorporated by reference herein.

The botulinum toxin referred to herein above, may be part of a composition or pharmaceutical composition. This pharmaceutical composition may contain additional pharmaceutically active components. "Pharmaceutical composition" is a formulation in which an active ingredient for use as a medicament or diagnostic is contained or comprised. Such pharmaceutical composition may be suitable for diagnostic or therapeutic administration (i.e. by intramuscular or subcutaneous injection) to a human patient. The pharmaceutical composition may be lyophilized or vacuum dried, reconstituted, or in solution. When reconstituted it is preferred that the reconstituted solution is prepared adding sterile physiological saline (0.9% NaCl).

Such composition may comprise additional components such as a pH buffer, excipient, diluent, cryoprotectant and/or stabilizer.

"pH buffer" refers to a chemical substance being capable to adjust the pH value of a composition, solution and the like to a certain value or to a certain pH range.

"Stabilizing", stabilizes" or "stabilization" means that the active ingredient, i.e., the botulinum toxin complex or neurotoxic component in a reconstituted or aqueous solution pharmaceutical composition has greater than about 20%, 30%, 40%, 50%, 60%, 70%, 80%, 90%, and up to about 100% of the toxicity that the biologically active neurotoxic component had prior to being incorporated into the pharmaceutical composition. The activity of the preparation may be determined as described elsewhere herein.

"Cryoprotectant" refers to excipients which result in the active ingredient, i.e., a botulinum toxin complex or neurotoxic component in a reconstituted or aqueous solution pharmaceutical composition has greater than about 20%, 30%, 40%, 50%, 60%, 70%, 80%, 90%, and up to about 100% of the toxicity that the biologically active neurotoxic component had prior to being freeze-dried in the pharmaceutical composition. The activity of the preparation may be determined as described elsewhere herein.

Examples of such stabilizers are gelatin or albumin, in one embodiment of human origin or obtained from a recombinant source. The stabilizers may be modified by chemical means or by recombinant genetics. In one embodiment of the present invention, it is envisaged to use alcohols, e.g., inositol, mannitol, as cryoprotectant excipients to stabilize proteins during lyophilization.

In another embodiment of the present invention, the stabilizer may be a non-proteinaceous stabilizing agent comprising hyaluronic acid or polyvinylpyrrolidone or polyethyleneglycol or a mixture of two or more thereof.

In even another embodiment of the present invention, the pharmaceutical composition may comprise the botulinum toxin complex or neurotoxic component and a hyaluronic acid or a polyvinylpyrrolidone or a polyethleneglycol, such composition being optionally pH stabilized by a suitable pH buffer, in particular by a sodium acetate buffer, and / or a cryoprotectant polyalcohol.

Whether or not the pharmaceutical composition comprises, beside the botulinum toxin, additional components such as albumin, hyaluronic acid, a polyvinylpyrrolidone and/or a polyethyleneglycol stabilizer, the pharmaceutical composition retains its potency substantially unchanged for six month, one year, two year, three year and/or four year periods when stored at a temperature between about +8°C. and about -20°C. Additionally, the indicated pharmaceutical compositions may have a potency or percent recovery of between about 20% and about 100% upon reconstitution.

A pharmaceutical composition within the scope of the present invention may include the botulinum toxin one or more additional components. The pharmaceutical compositions disclosed herein, has a pH of between about 4 and 7.5 when reconstituted or upon injection, in one embodiment between about pH 6.8 and pH 7.6 and in another embodiment between pH 7.4 and pH 7.6. Generally, the pharmaceutical composition of the present invention comprises neurotoxic component in a quantity of about 6pg to 30 ng. In one embodiment the neurotoxic component has a biological activity of 50 to 250 LD₅₀ units per ng neurotoxic component, as determined in a mouse LD₅₀ assay. In one embodiment the neurotoxic component has a biological activity of about 150 LD₅₀.

The pharmaceutical composition of the present invention may comprise a botulinum toxin, and a hyaluronic acid. The hyaluronic acid stabilizes the botulinum toxin. The pharmaceutical compositions disclosed herein may have a pH of between about 4 and 7.5 when reconstituted or upon injection. The hyaluronic acid in the instant pharmaceutical composition is in one embodiment combined with the instant neurotoxic component in a quantity of 0.1 to 10 mg, especially 1 mg hyaluronic acid per ml in a 200 U/ml botulinum toxin solution. In another embodiment the subject solution also contains a 1-100 mM, especially 10 mM sodium acetate buffer.

In yet another embodiment, the composition may contain a polyalcohol as cryoprotectant. Examples of polyalcohols that might be used include, e.g., inositol, mannitol and other non-reducing alcohols.

In particular those embodiments of the present invention's pharmaceutical composition not comprising a proteinaceous stabilizer, in one embodiment do not contain trehalose or maltotriose or related sugar or polyhydroxy compounds which are sometimes used as cryoprotectants.

The polyvinylpyrrolidone in the instant pharmaceutical composition is in one embodiment combined with the instant neurotoxic component in a quantity of 10 to 500 mg, especially 100 mg polyvinylpyrrolidone per ml in a 200 U/ml botulinum toxin solution. In another embodiment, the subject solution also contains a 1-100 mM, especially 10 mM sodium acetate buffer.

The polyethyleneglycol in the instant pharmaceutical composition is in one embodiment combined with the instant neurotoxic component in a quantity of 10 to 500 mg, especially 100 mg polyethyleneglycol per ml in a 200 U/ml botulinum toxin solution. In another embodiment the subject solution also contains a 1-100 mM, especially 10 mM sodium acetate buffer.

Thus, the instant invention encompasses in yet another embodiment a botulinum toxin formulated in a pharmaceutical composition, which contains a hyaluronic acid stabilizer or a polyvinylpyrrolidone stabilizer or a polyethyleneglycol stabilizer. Additionally, the pharmaceutical composition may contain a sodium acetate buffer system and/or an alcoholic cryoprotectant.

In one embodiment the formulation comprises a hydrophilic polymer, a mixture of a polyalcohol and a sugar, wherein the weight ratio of polyalcohol to sugar is from 2:1 to 5:1 (wt-%), a detergent, wherein said formulation is free of stabilising proteins. The hydrophilic polymer within said formulation may be selected from e.g. hyaluronic acid and/or polyvinylpyrrolidone.

Both the botulinum toxin complex formulations as well as neurotoxic component formulations are suitable for the methods disclosed herein, however, using formulations of the neurotoxic component as described above allows significantly to increase the frequency of treatment due to the reduced amount of accompanying proteins and/or its diffusion characteristics.

It has been found that administration of a therapeutic effective amount of botulinum toxin into striatal brain tissue lead to a significant reduction of symptoms of movement disorders, such as Parkinson's disease symptoms (cf. examples).

The term "movement disorder" as mentioned herein encompasses any disorder as a result of hypercholinergic activity in the striatum. Examples for such movement disorders are idiopathic Parkinson's disease, drug-induced Parkinson's disease and Parkinson-plus syndromes.

Parkinson's disease results in loss of dopaminergic innervation to the striatum (and other basal ganglia) and a cascade of subsequent consequences. *Inter alia* to an increased release of acetylcholine by interneurons, thereby distorting network function and inducing structural changes that undoubtedly contribute to the symptoms.

Drug induced Parkinson's disease is an induction of a loss of dopaminergic innervation by certain drugs or toxins, i.e. MPTP (1-methyl-4-phenyl-1,2,3,6-tetrahydropyridine). Such a drug induced Parkinson's disease might be reversible or irreversible.

Parkinson-plus syndromes describe a group of several primary neurodegenerative disorders which have parkinsonian features, such as bradykinesia, rigidity, tremor, and gait disturbances, in common. These neurologic conditions are associated with complex clinical presentations that reflect degeneration in various neuronal systems. However, because of the common parkinsonian features, the disorders have been collectively named Parkinson-plus syndromes. Although separate clinical syndromes have been identified, modern immunocytochemical techniques and genetic findings suggest many underlying similarities broadly grouped into 2 types: synucleinopathies and tauopathies.

Patients with Parkinson-plus syndromes typically have a worse prognosis than those with Parkinson disease (PD), and Parkinson-plus syndrome responds poorly to the standard anti-Parkinson treatments. An adequate response to treatment in a patient with parkinsonian symptoms may indicate that a Parkinson-plus syndrome is developing, and searching for the signs and symptoms of degeneration in other neuronal systems is important.

The term "hyperactive inhibitory cholinergic neurons in the striatum", as used herein, relates to neurons, which are characterized by an unusually high amount of acetylcholine release into the synaptic cleft. "Unusually high" relates to an increase of up to 5%, up to 50% or more with respect to a reference activity which may be obtained, for example, by comparing the release of inhibitory cholinergic neurons of the area to be treated with the release of inhibitory cholinergic neurons which are not in a hyperactive state. In one embodiment such hyperactive inhibitory cholinergic neurons might be identified by their acetylcholine-release with methods well known in the art (e.g. using a chemiluminescent agents or antibody stain). Another example for a method of performing the required measurements can be found in Tossman et al., "The effect of apomorphine and pergolide on the potassium-evoked overflow of GABA in rat striatum studied by microdialysis", Eur. J. Pharmacol., 1986; 123: 295-298. "Up to 5%" means herein, for example, about 1 % to about 5%.

The term "about" as used in the context of the present invention means "approximately" or "nearly". In the context of numerical values, without committing to a strict numerical definition, the term may be construed to estimate a value that is +/-10% of the value or range indicated.

The terms "striatum" and "striatal tissue" as defined herein comprise all structures of the corpus striatum (striated body) comprising the putamen and the caudate nucleus, sensorimotor striatum and associative striatum. To separate these territories from other areas of the brain well known anatomical and/or functional evaluations, as well as calbindin immunochemistry may be used.

The term "mammal" as used herein refers to a class of vertebrate animals whose name is derived from their distinctive feature, mammary glands, with which they feed their young. They are also characterized by the possession of sweat glands, hair, three middle ear bones used in hearing, and a neocortex region in the brain. The term "mammals" also encompasses humans.

The term "patients" herein encompasses a "mammals" i.e. humans or animals who have never been exposed to botulinum toxin as well as patients who have been exposed to botulinum toxin. The latter patients may have developed antibodies directed against the botulinum toxin complex or its components. Such antibodies may be neutralizing antibodies.

In another embodiment of the present invention, the patient is a human, who has been treated with *Botulinum* toxin but who complains about a decrease of the treatment effect and who requires treatment before expiry of 3 months after the treatment.

Such decreases of the therapeutic effect can be monitored by treatment calendars in which the patient records the severity of his disorder on a day-to-day basis (such treatment calendars are, for example, distributed by Merz Pharmaceuticals).

The term "administration" as used herein encompasses any path by which the botulinum toxin or botulinum toxin composition is brought into contact with the body. In one embodiement such an administration is done by injection, in another embodiment by perfusion or infusion.

In one embodiment the botulinum toxin or botulinum toxin formulation is applied via a micro-catheter-like device, enabling the skilled person to administer the formulation without the need of a head surgery. A non-limiting example for such an device can be found in DE10/2006/020402 (US2007/0265551). Such devices would also enable the skilled person to reapply the botulinum toxin if needed.

The administration of the botulinum toxin or botulinum toxin formulation might also be facilitated by an delivery device e.g. a convection-enhanced delivery device (CED) (cf. e.g. WO 2009/023601).

Micro-catheter-like devices or convection-enhanced delivery devices can be used for a "long-term application" of the botulinum toxin when needed. The term "long-term application" herein refers to treatment periods of 1, 2, 3, 4, 5, 6, 7, 8, 9, 10, 11, 12 months up to 1, 2, 3, 4, 5, 6, 7, 8, 9, 10 years.

As used throughout the specification of the present invention, the term "total amount administered per treatment" refers to the total dosage and means the sum of botulinum toxin or botulinum toxin composition applied to a patient during a single treatment. A single treatment may involve one or more administrations. The amount used for treatment is dependent on a number of parameters, which are known in the art. Such parameters include for example the serotype of the botulinum toxin and a number of patient specific factors. It is envisaged by the teaching of the present invention that a single treatment may be subdivided into two or more treatment sessions during which the above mentioned total amount of botulinum toxin is administered. This will be particularly the case if large amounts of botulinum toxin are to be administered.

The above-mentioned subdivision of administrations encompasses the administration into different parts of the striatal tissue ("local subdivisions") e.g. a first injection into the *putamen* and a second injection into the *caudate nucleus.* In another embodiment the local subdivision comprises a first administration into a caudal area of the striatum and at least a second administration into e.g. ventral area of the striatum.

In one embodiment the substructure of administration is the *putamen*, however, in another embodiment also administration into the *nucleus caudatus*, especially its latero-inferior parts, in yet another embodiment the administration is directed to the *sensorimotor striatum* or *associative striatum.*

In another embodiment adjacent areas of the striatal regions mentioned above, are administered in such a way, that diffusion effects result in a reduction of hyperactive inhibitory cholinergic neurons in the striatal tissue. In yet another embodiment the administration into striatal and/or adjacent regions is performed in such a way, that no hyperactive inhibitory cholinergic neurons of other brain regions selected from the group comprising thalamus, globus pallidus, pontine region and mesopontine region are affected.

In another embodiment the subdivisions may be separated by time ("temporal subdivisions") i.e. in accordance with the present invention, a first and a second and a subsequent treatment session may be scheduled one day after a preceding treatment session. The term "one day after" means e.g. 1, 2, 3, 4, 5, 6, 7, 8, 9 or 10 days or 1, 2, 3, 4, 5, 6, 7, 8, 9, 10, 11 or 12 weeks. However, it is also envisaged by the teaching of the present invention that the second treatment is scheduled only few hours after the first treatment, e.g. 2, 3, 4, 5, 6, 7, 8 up to 24 hours later. With respect to further administration schemes reference is made to WO 2008/000490, which is fully incorporated.

In yet another embodiment, the second (subsequent) treatment is carried out at a point in time when the efficacy of the first (previous) treatment begins to decline.

In another embodiment a combination of a local subdivision as well a temporal subdivision of treatment is envisaged i.e. treating different striatal tissues at subsequent time-points.

In one embodiment of the present invention, a second and any local and/or temporal subdivisional treatment is performed in order to improve the treatment effect of the first treatment. This will allow administration of appropriate botulinum toxin doses more efficiently. For example, in a first treatment session a suboptimal dose of the botulinum toxin may be administered. Should the patient's disease symptoms not sufficiently respond, more botulinum toxin may be administered in a second or in subsequent treatment session(s). Therefore, in view of the reduced risk associated with the methods of the present invention, a number of treatment sessions may be used in order to approach the optimal dose necessary to effectively treat a patient.

In one embodiment the administration is via injection.

In another embodiment, the first injection is used to determine the exact muscle or gland affected by the corresponding striatal region, whereas the subsequent injections are used to increase the denervating effects until the desired improvement of symptoms is reached. For example if a tremor of the left arm is treated in a patient, one or more injections with very low dose might be used to localize the striatal area responsible for the control of the muscles of the left arm, whereas, after said area is identified, subsequent injections with higher dosages are applied until the tremor of the arm is decreased or stopped.

In another embodiment the subsequent treatment is repeated until at least one of the targeted symptoms of Parkinson's disease is alleviated to a tolerable extend. Symptoms which might be targeted are selected but not limited to muscle rigidity, stiffness, difficulty to bend arms or legs, instable, stooped or slumped-over posture, loss of balance, changes in walking pattern, shuffling walk, slow movements, difficulty beginning to walk, difficulty initiating any voluntary movement, small steps followed by the need to run to maintain balance, freezing of movement when the movement is stopped, inability to resume, movement muscle aches and pains (myalgia), shaking, tremors, changes in facial expression, reduced ability to show facial expressions, inability to close mouth, reduced rate of blinking, voice/speech changes, slow speech, difficulty of speaking, loss of fine motor skills, difficulty of writing, difficulty of eating.

The injection itself may be guided by three-dimensional stereotactic magnetic resonance imaging and electrophysiological guidance, as is known in the prior art (cf. e.g. Harlz et al., "A quick and universal method for stereotactic visualization of the subthalamic nucleus before and after implantation of deep brain stimulation electrodes, Stereotact. Funct. Neurosurg., 2003; 80: 96-101). Furthermore, preliminary MRI (Magnetic resonance imaging) and/or NMRI (nuclear magnetic resonance imaging) is used to define the orientation of the external bony landmarks. CT (computer tomography), supplemented with cerebral ventriculography, are used for guidance to target the desired brain area.

The above specified (and claimed) modes of administration of the medicament as used within the present invention belong usually to the activities of the physicians treating patients. However, the mode of administration can be also part of the manufacture of the medicament in that e.g. the package of the medicament contains a specifically adapted leaflet with instructions to the physician and/or the patient and/or the package is specifically adapted to allow the mode of administration according to the present invention.

With such a treatment regimen, a stable quality of life for the patients can be achieved.

The determination of the parameter "stable quality of life" for the patients is exemplarily described hereinunder by reference to one condition to be treated according to the present invention, namely Parkison's disease, on the basis of the so-called Unified Parkinson's Disease Rating Scale (UPDRS). The Unified Parkinson's Disease Rating Scale is a rating scale used to follow the longitudinal course of Parkinson's disease.

It is made up of the following sections:
- Mentation, behavior, and mood;
- Activities of daily living;
- Motor function;
- Complications of therapy;
These are evaluated by interview and clinical observation. Some sections require multiple grades assigned to each extremity. A total of 199 points is possible. 199 represents the worst (total) disability, 0 points represent no disability. Each clinicians and researchers alike use the UPDRS and the motor section in particular to follow the progression of a person's Parkinson's disease. A "stable-life quality" is achieved by < 20-30% of the values of UPDRS (i.e. less than 40 to 80 points out of 199 of UPDRS).

The UPDRS_{actual} is determined before the first injection resulting in a base-line level and after the respective (re)-injection of the medicament and up to 3 weeks thereafter, respectively.

Finally, the observed differences between the UPDRS level at the day of a re-injection treatment and the baseline UPDRS_{actuat} level will be analyzed to investigate if there is any improvement over time of the quality of life level at the time of re-injection (expected time of waning of treatment effect).

The term "effective amount" means an amount of botulinum toxin, which, after administration, results in a partial or complete removal of disease symptoms. It depends greatly on the patients physical condition, the muscle or gland to be targeted, the magnitude of the symptoms to be treated and the sensitivity of the patient to the treatment. Effective amounts are generally in the range of 0.01 up to 200 MU, but also doses of up to 500 MU may be used in very rare cases. In one embodiment a range of 1 to 50 MU is applied, in another embodiment a range of 0.1 to 20 MU, in yet another embodiment a range of 0.01 to 10 MU is applied. When high doses of botulinum toxin are to be administered to a patient, it may be beneficial to split the treatment into more than one treatment session as also described hereinunder elsewhere. The term "more than one treatment session" means e.g. 2, 3, 4, 5, 6, 7, 8, 9 or 10 treatment sessions.

The biological activity is commonly expressed in Mouse Units (MU). As used herein, 1 MU is the amount of botulinum toxin, which kills 50% of a specified mouse population after intraperitoneal injection, i.e. the mouse *i.p.* LD₅₀ (Schantz & Kauter, 1978). The terms "MU" and "Unit" or "U" are interchangeable. Alternatively, the biological activity may be expressed in Lethal Dose Units (LDU)/ng of protein (i.e. botulinum toxin complex or neurotoxic component). The term "MU" is used herein interchangeably with the terms "U" or "LDU" or "LD₅₀".

Based on experience in the animal model the amount of locally applied neurotoxic component might be for example at least 1 ng. The actual dose will depend on the mentioned patient-dependent factors and on the clinical response of the patient. The concentration of the neurotoxic omponent in the buffer solution to be applied will be for example about 10 nM.

### EXAMPLES

The following examples are provided by means of illustration only, and are not intended to be limiting.

### Example 1: Botulinum Toxin injection in a 6-OHDA-induced animal model

For the animal model of Parkinson's disease unilateral 6-hydroxy-dopamine(6-OHDA)-lesions were placed in the right median forebrain bundle by injection of 8 µg of the toxin 6-OHDA. This deletes highly sensitive dopaminergic neurons of the substantia nigra mimicking the characteristics of Parkinson's disease.

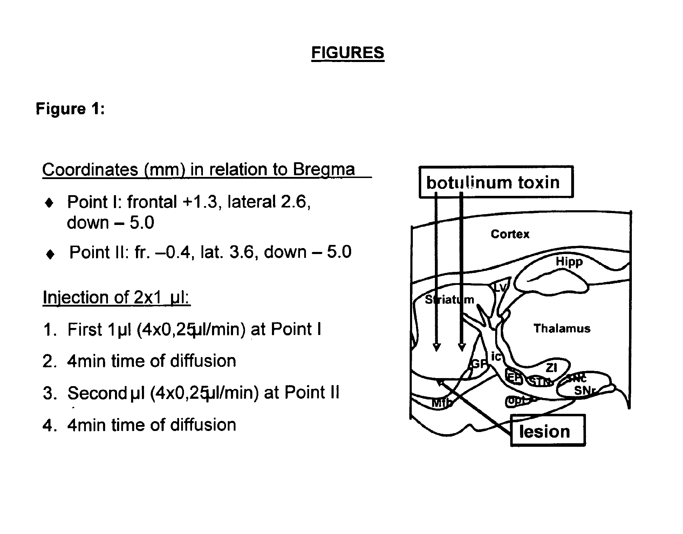

Six weeks after the treatment 2x 1 µl botulinum toxin A in different concentrations was injected into the striatum (Figure 1). The coordinates of the injection-sites are indicated with respect to the bregma (intersection point of *sutura coronalis* and *sufura sagittalis* of the skull) (cf. description of figure 1).

### Example 2: Rotation test

To quantify the motoric dysfunction the apomorphine-induced rotation test of unilateral hemiparkinson animals were used (cf. example 1). Unilateral lesion of the right median forebrain bundle leads to anticlockwise rotation (i.e. positive values of rotation per minute) of the animals after intraperitoneal administration of apomorphine. The rate of rotational frequency correlates with the severity of the deficit of motor function.

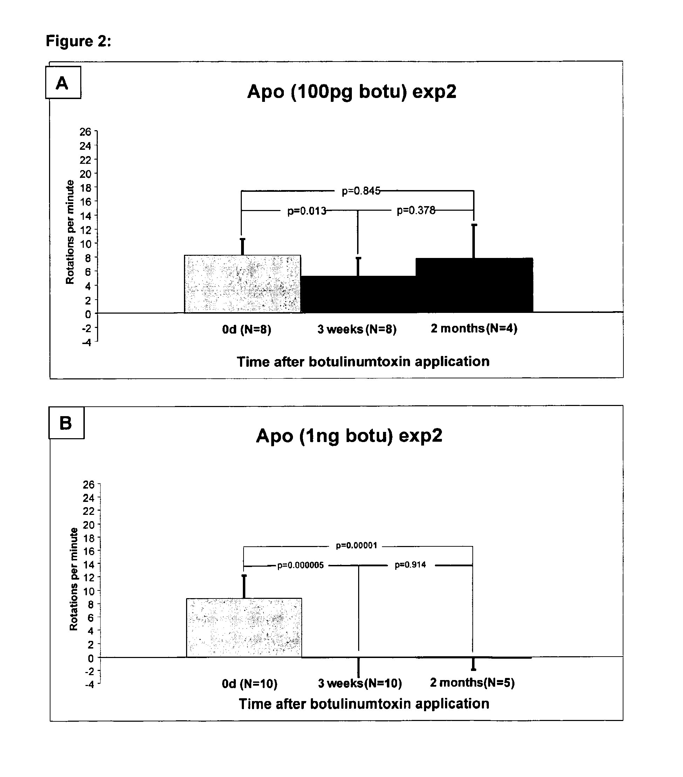

6-OHDA-rats as treated according to example 1 showed about 8 rotations per minute. Intrastriatal administration of botulinum toxin A in low dosages (100 pg, figure 2A) led to a transient reduction (≥ 3 weeks) of rotational frequency (reduction from 8 to 5 rotations per minute). High dosage administration (1 ng, figure 2B) led to a long-lasting (> 2 months) complete annihilation of pathological rotation (Figure 2). Side-effects, like for example delayed growth or behavioural anomalies, could not be observed.

### Example 3: Botulinum toxin administration into healthy animals

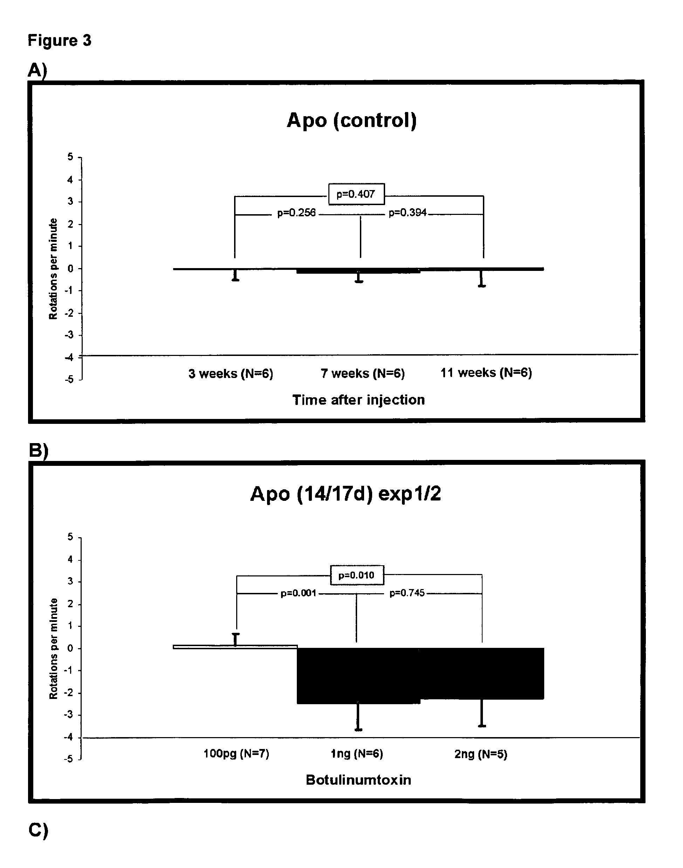

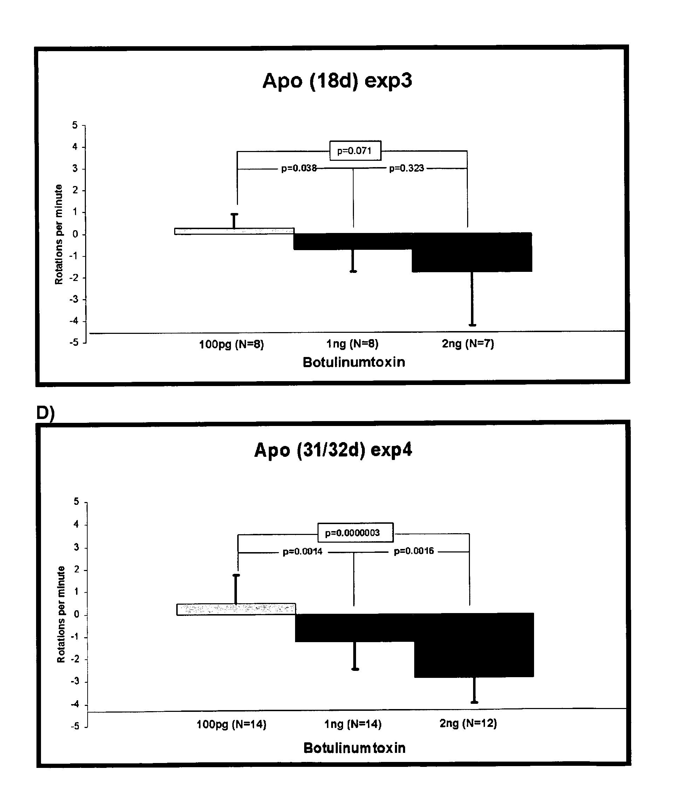

The injection of high botulinum toxin dosages (1-2 ng) into healthy control animals lead to a clockwise rotation (2-3 rotations per minute) (cf. Figure 3). This might be due to the fact that injection of apomorphine stimulates dopaminergic neurons simultaneously, whereas the unilateral injection of botulinum toxin leads to a reduced inhibitory activity of the cholinergic neurons of the striatum resulting in an asymmetric motor function.

Control animals, which were injected with phosphate-buffered saline and 0.1 % BSA (bovine serum albumin), showed no pathological rotation (Figure 3A).

### Example 4: Treatment of a Patient with Parkinson's disease

A male patient of 68 years shows a general bradykinesia and rigor of his right ellbows as well as several other symptoms of Parkinson's disease. Furthermore, the patient suffers from a strong tremor in his right arm and hand (nine beats per minute), preventing him to use his right hand for eating, writing etc.. The patient is treated with dopaminergic medicaments which lead to optical and acoustical hallucinations. A UPDRS - test without the treatment with dopaminergic medicaments results in 146 points. An area of hyperactive inhibitory cholinergic neurons in the ventrolateral part of the striatum is identified with standard methods. Using three-dimensional stereotactic magnetic resonance imaging and electrophysiological guidance a low dose of XEOMIN^{®} is injected into the identified ventrolateral area of the striatal tissue in order to locate the specific areas corresponding to the right arm tremor and ellbow rigor. After a mild reduction of tremor is noticed (3-4 beats per minute), a higher dose of XEOMIN^{®} is injected subsequently until no tremor and ellbow rigor is detected. A subsequent UPDRS - test one week after the treatment results in 78 points. The patient remains stable for 5 months until another treatment with XEOMIN^{®} becomes necessary. Furthermore, the treatment with dopaminergic medicaments can be reduced thereby decreasing hallucination side-effects.

## Claims

1. *Clostridium botulinum* toxin for the treatment of a movement disorder caused by or associated with hyperactive inhibitory cholinergic neurons of the striatum of a mammal, comprising administering an effective amount to said mammal, wherein the toxin is administered into striatal tissue of the mammal's brain.

2. *Clostridium botulinum* toxin of claim 1, wherein the mammal is human.

3. *Clostridium botulinum* toxin of claim 1 or 2, wherein the movement disorder comprises at least one of the symptoms selected from muscle rigidity, tremor, a slowing of physical movement (bradykinesia) and a loss of physical movement (akinesia).

4. *Clostridium botulinum* toxin of any of the preceding claims, wherein the movement disorder is Parkinson's disease.

5. *Clostridium botulinum* toxin of any of the preceding claims, wherein the striatal tissue is selected from putamen, nucleus caudatus, sensorimotor striatum and associative striatum or any combination thereof.

6. *Clostridium botulinum* toxin of any of the preceding claims, wherein the administration is split into more than one local or temporal, or local and temporal subdivisions.

7. *Clostridium botulinum* toxin of claim 6, wherein the temporal subdivision is an administration at an interval of less than three months, the interval comprising a first injection and at least one second injection, wherein the amount administered in the second injection can be lower, higher or identical to the amount administered in the first injection.

8. *Clostridium botulinum* toxin of any of the preceding claims, wherein the *Clostridium botulinum* toxin is selected from the group consisting of type A, B, C, D, E, F, G or a mixture thereof.

9. *Clostridium botulinum* toxin of any of the preceding claims, wherein the *Clostridium botulinum* toxin is the neurotoxic component devoid of any other protein component of the *Clostridium botulinum* toxin complex.

10. The neurotoxic component of claim 9, wherein the neurotoxic component is serotype A.

11. *Clostridium botulinum* toxin of any one of the preceding claims, wherein the administration is selected from the group of injection, perfusion and infusion including application by infusion pumps.

## Amended claims

### Amended claims in accordance with Rule 137(2) EPC.

**1.** *Clostridium botulinum* toxin for the use in the treatment of Parkinson's disease, comprising administering an effective amount to said mammal, wherein the toxin is administered into striatal tissue of the mammal's brain.

**2.** *Clostridium botulinum* toxin for the use according to claim 1, wherein the mammal is human.

**3.** *Clostridium botulinum* toxin for the use according to claim 1 or 2, wherein the movement disorder comprises at least one of the symptoms selected from muscle rigidity, tremor, a slowing of physical movement (bradykinesia) and a loss of physical movement (akinesia).

**4.** *Clostridium botulinum* toxin for the use according to any of the preceding claims, wherein the striatal tissue is selected from putamen, nucleus caudatus, sensorimotor striatum and associative striatum or any combination thereof.

**5.** *Clostridium botulinum* toxin for the use according to any of the preceding claims, wherein the administration is split into more than one local or temporal, or local and temporal subdivisions.

**6.** *Clostridium botulinum* toxin for the use according to claim 5, wherein the temporal subdivision is an administration at an interval of less than three months, the interval comprising a first injection and at least one second injection, wherein the amount administered in the second injection can be lower, higher or identical to the amount administered in the first injection.

**7.** *Clostridium botulinum* toxin of any for the use according to the preceding claims, wherein the *Clostridium botulinum* toxin is selected from the group consisting of type A, B, C, D, E, F, G or a mixture thereof.

**8.** *Clostridium botulinum* toxin of any for the use according to the preceding claims, wherein the *Clostridium botulinum* toxin is the neurotoxic component devoid of any other protein component of the *Clostridium botulinum* toxin complex.

**9.** The neurotoxic component for the use according to claim 8, wherein the neurotoxic component is serotype A.

**10.** *Clostridium botulinum* toxin for the use according to any one of the preceding claims, wherein the administration is selected from the group of injection, perfusion and infusion including application by infusion pumps.
